# EUROPEAN PATENT SPECIFICATION

(11) **EP 3 689 305 B1**
(45) Date of publication and mention of the grant of the patent: **15.03.2023**
(21) Application number: 20154906.0
(22) Date of filing: 31.01.2020
(51) Int. Cl.: A61F 13/00, A61F 13/02

(54) **NEGATIVE PRESSURE WOUND DRESSING AND NEGATIVE PRESSURE WOUND TREATMENT DEVICE**
UNTERDRUCKWUNDAUFLAGE UND UNTERDRUCKWUNDBEHANDLUNGSVORRICHTUNG
PANSEMENT PAR PRESSION NÉGATIVE ET DISPOSITIF DE TRAITEMENT DE PLAIES PAR PRESSION NÉGATIVE

(30) Priority: 01.02.2019 TW 108201730 U
(43) Date of publication of application: 05.08.2020
(73) Proprietor: Evophancie Biotech Ltd., New Taipei City 248 (TW)
(72) Inventor: LU, Chi-Hsiang, 248 New Taipei City (TW); HONG, Jun-Wei, 248 New Taipei City (TW); LIN, Shang-Ru, 248 New Taipei City (TW)
(74) Representative: Epping - Hermann - Fischer

(56) References cited:
- EP-A1- 3 124 055
- EP-A1- 3 315 145
- WO-A1-01/85248
- WO-A1-2015/010445
- CN-U- 203 408 345
- US-A1- 2005 186 260
- US-A1- 2009 209 897
- US-A1- 2013 149 368
- US-A1- 2018 353 339

## Description

### BACKGROUND

### 1. Technical Field

The present application relates to a wound dressing, and more particularly to a wound dressing for a negative pressure wound therapy.

### 2. Description of the Related Art

Negative Pressure Wound Therapy (NPWT) is an auxiliary treatment for wound healing. It is suitable for many acute and chronic wounds, including orthopedics, soft tissues, skin grafts, pressure sores, lower extremity venous ulcers, diabetic foot, surgery infection, postoperative wounds, etc. The NPWT is placed in a cleaned wound with a foam corresponding to a shape of the wound, and the foam is in full contact with the wound surface. Since there are many holes in the foam, a vacuum pump can be used to apply a negative pressure to the wound filled with the foam. The pressure causes pus and infectious substances in the wound to be discharged through the holes in the foam, and also attracts the tissue fluid into the foam to maintain the moist therapy environment of the wound, so as to promote blood microcirculation around the wound and the growth of the new tissue. Thus, the effect of accelerating wound healing is achieved.

However, the foam in NPWT, especially those using hydrophobic polyurethane foam, has high adhesion to the wound, and the new tissue easily grows into the holes in the foam, and the foam must be replaced about every two to three days. If the infection of the wound is serious, the frequency of replacing the foam may be more according to the physician's diagnosis. Every time the foam is changed, due to the adhesion of the wound and the growth of the new tissue, patients often suffer severe pain, and there is also a secondary injury to the wound, which is a serious problem in clinical application.

US 2018/0353339 A1 discloses a composite dressing for improved granulation and recued maceration with negative-pressure treatment, and the composite dressing may include a polymer of carboxymethyl cellulose or oxidized regenerated cellulose. However, US 2018/0353339 A1 is silent on a bio-fiber membrane made by bacteria.

CN 203408345 U and WO 2015/010445 A1 both disclose a disposable portable negative-pressure wound and scar therapy system, but fail to disclose a bio-cellulose film comprising bio-celluloses made by bacteria.

EP 3315145 A1 discloses a multi-layer wound care products including an intermediate bacteria-absorbing layer, and the multi-layer wound care products can be used for negative-pressure therapy. Nevertheless, the intermediate bacteria-absorbing layer thereof is placed on the intermediate layer and cannot be in direct contact with the wound. Despite that EP 3315145 A1 discloses that the multilayered wound care product can be cut or perforated in the bottom layer for allowing the intermediate bacteria-absorbing layer to partially contact the wound, it is still unlikely for the bacteria-absorbing layer to prevent the wounds from sticking to the wound care product.

Therefore, how to avoid the adhesion of foam to the wound and to avoid the growth of new tissue into the holes in the foam is keenly aimed to be achieved in the clinical and industrial fields.

### SUMMARY

To solve the above problems, the present application provides a negative pressure wound dressing according to claim 1, and a negative pressure wound treatment device according to claim 10. Preferred embodiments are set forth in the subclaims.

The bio-cellulose film refers to a microorganism, for example, bacteria of at least one genus selected from the group consisting of *Gluconacetobacter, Acetobacter, Rhizobium, Sarcina, Pseudomonas, Achromobacter, Alcaligenes, Enterobacter, Azotobacter* and *Agrobacterium,* which uses the carbon source in the culture medium to form *β*-1,4-glucan chain in the cell, and then secrets bio-cellulose extracellularly to form the bio-cellulose film.

In one embodiment, the bio-cellulose film is formed by culturing bacteria in a culture medium having a carbon source, a nitrogen source, and, optionally, a yeast extract. The culture is, for example, a static culture. The carbon source includes, for example, glucose, mannitol, molasses, fructose, sucrose, etc. The nitrogen source is such as peptone. Further, a weight ratio of the carbon source, the nitrogen source, and the yeast extract may be 60:6:6 to 15:3:0; or 60:6:6 to 15:3:0.5; or 60:6:6 to 15:3: 1; or 60:6:6 to 15:3:2; or 60:6:6 to 15:3:3; or 60:6:6 to 15:3:4; or 60:6 :6 to 15:3:5.

According to the present invention, the three-dimensional reticular structure has a plurality of backbone fibers parallel to each other and a plurality of interlayered fibers interwoven with any two adjacent backbone fibers, wherein the plurality of backbone fibers and the plurality of interlayered fibers are interwoven with each other to form nano-sized pores.

In another embodiment, the backbone fibers and the interlayered fibers are made of bio-celluloses, and a diameter of the backbone fiber is greater than or equal to a diameter of the interlayered fiber.

In one embodiment, the bio-cellulose has a diameter of 20 to 100 nm.

According to the present invention, the bio-cellulose film has a bio-cellulose content of 0.0013 to 0.0018 g/cm² per unit area.

In one embodiment, the negative pressure wound dressing comprises a drug. For example, the drug for promoting wound healing, e.g., antibacterial or anti-inflammatory, and promotion of cell growth, includes but is not limited to, at least one selected from the group consisting of antibiotics, antibacterial agents, growth factors, fibrin, vasodilators, angiogenesis promoters, antioxidants, and anti-inflammatory agents. More specifically, the drug is contained in the foam, in the bio-cellulose film, or in both of the foam and the bio-cellulose film.

In one embodiment, the foam comprises at least one material selected from the group consisting of polyurethane foam, polyvinyl alcohol foam, polyether foam, polyester foam, polylactic acid foam, polyolefin foam, chitosan foam, cellulose foam, alginate foam, gelatin foam, and collagen foam.

In another embodiment, the foam has a multilayer structure, including, e.g., a contact layer, an absorption layer and a drainage layer, wherein the contact layer is in contact with the bio-cellulose film.

The present application also provides a negative pressure wound treatment device, comprising: the negative pressure wound dressing; a liquid delivery tube connected to the negative pressure wound dressing; and a negative pressure source.

In one embodiment, the negative pressure wound treatment device further comprises a sealing film, which is disposed on the negative pressure wound dressing and is used to cover a wound and a normal skin outside the wound.

In another embodiment, an opening is provided in the center of the sealing film and penetrates through two opposite surfaces of the sealing film, and one end of the liquid delivery tube extends through the opening to the foam of the negative pressure wound dressing.

In one embodiment, the negative pressure wound treatment device further comprises a liquid-collecting tank connected to the other end of the liquid delivery tube, and the liquid-collecting tank is connected to the negative pressure source through a connecting tube.

The negative pressure wound dressing of the present application is bonded to the surface of the foam through the bio-cellulose film. When performing the negative pressure wound treatment (NPWT), the bio-cellulose film exists between the foam filled in the wound and the wound surface. When changing the dressing, it can avoid the wound adhering to the material, and reduce the severe pain in the patient. Further, since the bio-cellulose film has a three-dimensional reticular structure and the three-dimensional reticular structure has nano-sized pores formed by interweaving backbone fibers and interlayered fibers, it has excellent gas permeability, and does not damage the negative pressure environment. On the other hand, the nano-sized pores of the bio-cellulose film and the pores of the foam can also carry drugs, which can release the drug on the surface of the wound while sucking out the pus and the infectious substance, so as to promote the healing of the wound.

### BRIEF DESCRIPTION OF THE DRAWINGS

FIG. 1 is a schematic diagram showing the structure of the negative pressure wound dressing in the present application;
FIGs. 2A and 2B are scanning electron micrographs (SEM) of the bio-cellulose film of the negative pressure wound dressing in the present application, wherein FIG. 2A is a photograph showing a three-dimensional reticular structure of a bio-cellulose film, and FIG. 2B is a photograph showing a side view of the bio-cellulose film;
FIG. 3 is a schematic view showing the foam of the negative pressure wound dressing in the present application having a multilayer structure; and
FIGs. 4A to 4D are schematic diagrams for implementing the negative pressure wound treatment device in the present application.

### DETAILED DESCRIPTION OF THE EMBODIMENTS

The examples of the present application are described below by way of embodiments, and those skilled in the art can readily appreciate the advantages and functions of the present application from the disclosure herein. The present application may be embodied or applied by other different embodiments. The details of the present specification may also be based on different opinions and applications, and various modifications and changes may be made without departing from the spirit of the present application. In addition, all ranges and values herein are inclusive and combinable. Any value or point falling within the ranges recited herein, such as any integer, may be the minimum or maximum value to derive the lower range and the like.

In the preparation of the negative pressure wound dressing in the present application, the foam is placed on a bottom surface or fixed in a container with a culture medium, and the liquid level of the culture medium is slightly higher than the foam. The foam is completely immersed in the culture medium, and statically culturing bacteria of at least one genus selected from the group consisting of genus *Gluconacetobacter, Acetobacter, Rhizobium, Sarcina, Pseudomonas, Achromobacter, Alcaligenes, Enterobacter, Azotobacter* and *Agrobacterium* in the culture medium, preferably, bacteria of one single genus such as genus *Gluconacetobacter* or *Acetobacter.* The culture time is preferably 24 to 96 hours. After the culture, the bio-cellulose film is formed on the foam, and the bio-cellulose film is bonded to the surface of the foam, wherein the culture medium includes a carbon source, a nitrogen source, and a yeast extract.

The projected area of the foam on the horizontal plane needs to be less than or equal to the solution surface area of the culture medium, so that the projected area of the bio-cellulose film, which formed by the gas-liquid interface, on the surface where the foam is bonded to the bio-cellulose film is greater than or equal to the projected area of the foam.

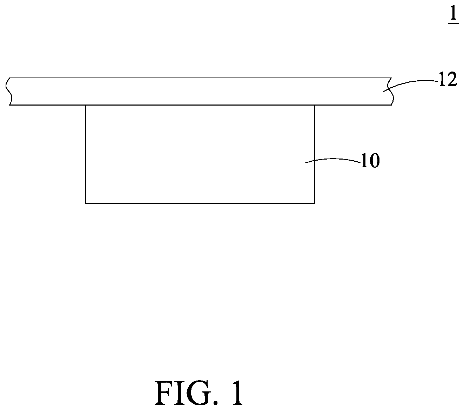

As shown in FIG. 1, the negative pressure wound dressing 1 of the present application comprises a foam 10; and a bio-cellulose film 12 disposed on the foam 10 to bond the bio-cellulose film 12 to the surface of the foam 10. Further, the projected area of the bio-cellulose film 12 on the surface where the foam 10 is bonded to the bio-cellulose film 12 is larger than the projected area of the foam 10. In another embodiment, the projected area of the bio-cellulose film 12 on the surface where the foam 10 is bonded to the bio-cellulose film 12 may be equal to the projected area of the foam 10.

In one embodiment, the culture medium comprises mannitol as a carbon source, peptone as a nitrogen source, and, optionally, a yeast extract, in a ratio by weight of 60:6:6 to 15:3:0. The culture medium is controlled to be acidic, for example, with a pH of between 0.5 and 6.5, the absorbance value reflecting the microbial concentration in the culture medium (wavelength setting of 620 nm) is controlled in the range of between 0.006 to 0.01, culture temperature is between 25 to 28 °C, and the culture time is 24 to 96 hours. After testing, the bio-cellulose film has a thickness of at least 0.3 to 0.6 mm, and the bio-cellulose in the bio-cellulose film has a diameter of about 20 to 100 nm. Further, the bio-cellulose film has a bio-cellulose content of from 0.0013 to 0.0018 g/cm² per unit area.

The static culture refers to the bacteria form a bio-cellulose in a non-woven manner on the surface of the culture medium; the bio-cellulose accumulates and extends from the surface of the culture medium toward the bottom of the container; then accumulated bio-cellulose gradually touches the surface of the foam; and finally, the bio-cellulose grows into the foam, such that the bio-cellulose film is bonded with the foam.

In addition, the container for static culture is preferably a flat container, which means that a height of the container is lower relative to a length and width of the container; through a lower container height and a wider growth area, the oxygen consumption of bacteria is controlled, so as to regulate the diameter of the bio-cellulose.

In one embodiment, the bio-cellulose film is formed by statically culturing bacteria of the genus *Gluconacetobacter* in a culture medium having mannitol, peptone, and yeast extract for fermentation.

The bacteria of the genus *Gluconacetobacter* are preferably *Gluconacetobacter xylinum,* which is characterized in that it is easy to control the degree of the thickness of the obtained bio-cellulose.

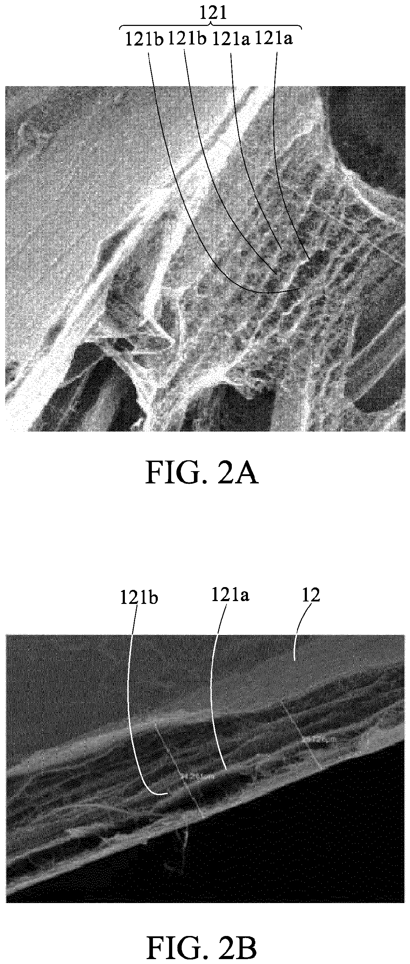

In one embodiment, the bio-cellulose film of the negative pressure wound dressing in the present application, which has a three-dimensional reticular structure, is as shown in FIG. 2A. The three-dimensional reticular structure 121 is formed by a plurality of bio-celluloses. Specifically, the three-dimensional reticular structure 121 has a plurality of backbone fibers 121a extending parallel to each other, or extending in the longitudinal direction or the width direction of the bio-cellulose film, and interlayered fibers 121b interwoven with the backbone fibers 121a. Thus, the interlayered fibers 121b are in the horizontal direction and in the vertical direction connected to any two adjacent backbone fibers 121a, so as to form a three-dimensional reticular structure 121. The backbone fibers 121a and the interlayered fibers 121b are all bio-cellulose. As shown, the diameter of the backbone fiber 121a is greater than or equal to the diameter of the interlayered fiber 121b. According to an embodiment of the present application, the surface of the bio-cellulose film formed in the longitudinal direction or in the width direction is a surface where the foam is bonded to the bio-cellulose film.

Referring to FIG. 2B, a side view of the bio-cellulose film 12 is shown, wherein the bio-cellulose film 12 also has a plurality of backbone fibers 121a that are parallel to each other or extend in the longitudinal or width direction of the bio-cellulose film 12, and the interlayered fiber 121b interwoven with the backbone fibers 121.

The foam in the present application is not limited, as long as it is an open cell and biocompatible foam. For example, Polyurethane foam, polyvinyl alcohol foam, polyether foam, polyester foam, polylactic acid foam, polyolefin foam, chitosan foam, cellulose foam, alginate foam, gelatin foam and collagen foam.

The foam is made of a single material or is made of a plurality of materials to form a foam having a composite structure. Further, the foam may have a multilayer structure, which refers to the layers each are continuous and the same in material, but have different physical properties such as density, porosity, permeability, compression ratio, etc. The physical properties can be obtained by adjusting various parameters during the manufacturing process. Also, the multilayer structure may refer to the layers each are different in materials, for example, a layered structure of polyurethane and polyvinyl alcohol. The layers each in the multilayer structure can provide different functions as desired, such as contact layers, absorption layers and drainage layers.

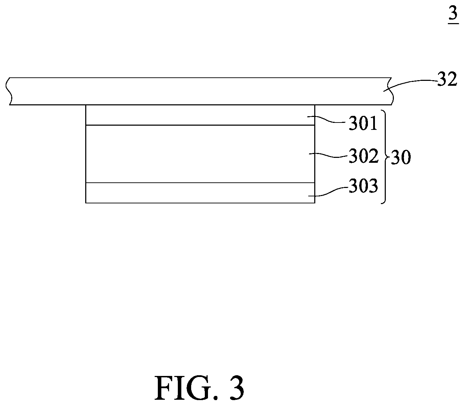

The foam of the negative pressure wound dressing in the present application can be a single layer structure, besides, in an embodiment as shown in FIG. 3, the negative pressure wound dressing 3 in the present application includes a foam 30 having a multilayer structure and the bio-cellulose film 32. The multilayer structure sequentially includes a contact layer 301, an absorption layer 302, and a drainage layer 303. The contact layer 301 contacts the bio-cellulose film 32. The contact layer 301 has larger pores than those pores formed in the absorption layer 302, so that parts of the bio-cellulose of the bio-cellulose film 32 can grow into the pores of the contact layer 301 to enhance the bonding between the bio-cellulose film 32 and the foam 30, and also the wound exudate can quickly enter the foam. The absorption layer 302 has better liquid absorption characteristics, and can temporarily accommodate the wound exudate. The drainage layer 303 has better exudate-transferring characteristics, such that the exudate absorbed by the foam can be drained to the liquid delivery tube of the negative pressure wound device.

After the bio-cellulose film is formed on and bond to the foam, the negative pressure wound dressing in the present application is formed. Then the obtained negative pressure wound dressing is washed to be neutral. The washing method is, for example, boiling with 1% of NaOH solution at 80 °C or higher for 15 minutes, then washed with water.

The washed negative wound dressing is then subjected to sterilization treatment. The sterilization treatment includes, but not limited to, autoclaving, gamma irradiation sterilization, ethylene oxide (EO) sterilization, and the like. Preferably, the autoclaving is used, for example, at 1.2 atm and 121 °C, for 20 minutes.

The negative pressure wound dressing of the present application may further comprise a drug. In one embodiment, the drug can be added after the washing procedure. Since the bio-cellulose film and the foam both have pores, the drug can be kept in the bio-cellulose film, in the foam, or in both of them. For example, the drug for promoting wound healing, such as antibacterial, anti-inflammatory, and promoting cell growth drugs can be selected. The drug includes but is not limited to, at least one selected from the group consisting of antibiotics, antibacterial agents, growth factors, fibrin, vasodilators, angiogenesis promoters, antioxidants, and anti-inflammatory agents.

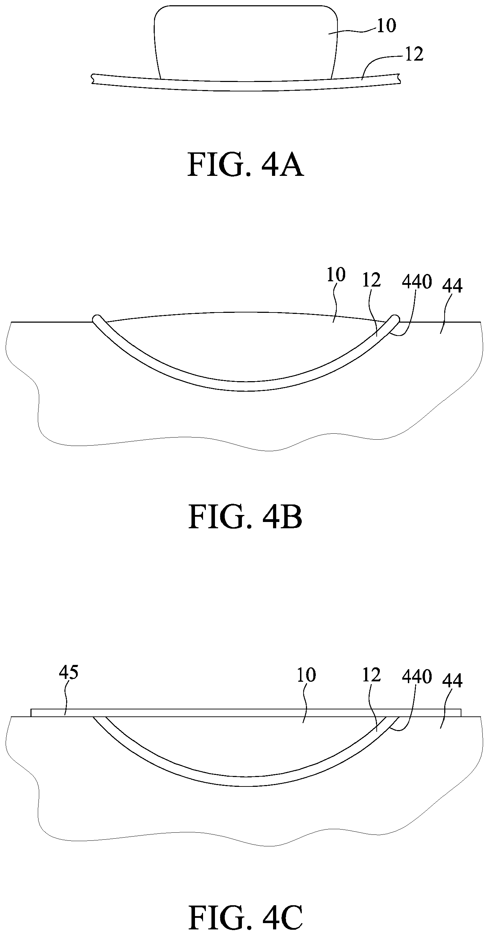

The negative pressure wound dressing of the present application can be applied to the negative pressure wound therapy (NPWT). In one embodiment, the negative pressure wound dressing and the sealing film, the liquid delivery tube, the liquid-collecting tank and the negative pressure source constitute a negative pressure wound treatment device. Specifically, the negative pressure wound treatment device of the present application can be implemented as shown in the schematic diagrams of FIG. 4A to 4G to treat a wound.

As shown in FIG. 4A, prepare the negative pressure wound dressing of the present application, which includes foam 10 and bio-cellulose film 12. Then, as shown in FIG. 4B, the negative pressure wound dressing of the present application is filled into the wound 440 of the injured site 44. The filling method is such that the bio-cellulose film 12 is directed toward the surface of the wound 440, and the foam 10 faces relatively outward.

The shape and volume of the foam 10 must be compatible with the wound 440. Before filling the foam 10 into the wound 440, the foam 10 can be appropriately trimmed so that the bio-cellulose film 12 is completely adhered to the surface of the wound 440 and the foam 10 is approximately flush with normal skin at injured site 44 outside the wound 440.

As shown in FIG. 4C, the sealing film 45 is completely covered with the negative pressure wound dressing. The area of the sealing film 45 needs to be larger than the wound 440, so that edges of the sealing film 45 can adhere to normal skin at injured site 44 outside the wound 440.

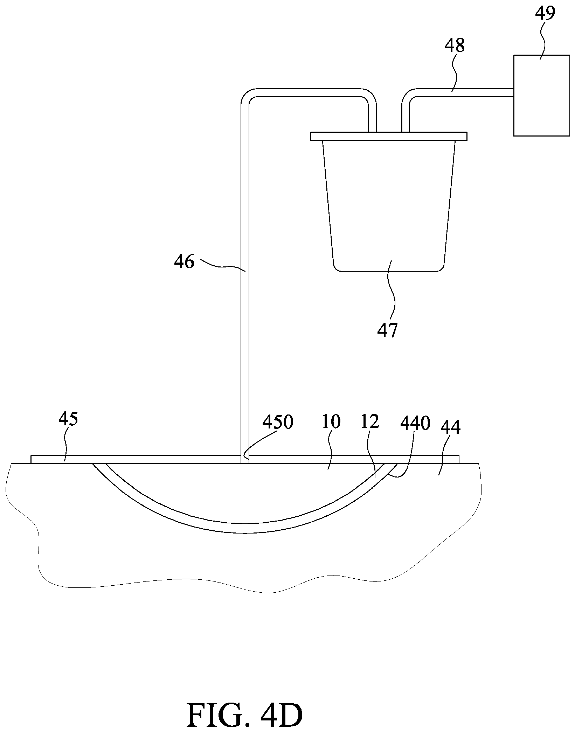

As shown in FIG. 4D, an opening 450 is formed in the center of the sealing film 45 and penetrates through two opposite surfaces of the sealing film 45, so that one end of the liquid delivery tube 46 can extend into the foam 10 of the negative pressure wound dressing through the opening 450. The other end of the liquid delivery tube 46 is connected to the liquid-collecting tank 47 and the liquid-collecting tank 47 is then connected to the negative pressure source 49, such as a pump through the connecting tube 48. After being sucked by the negative pressure source 49, pus and infectious substances in the wound 440 flow to the liquid-collecting tank 47 through the bio-cellulose film 12, the foam 10 and the liquid delivery tube 46.

Due to the barrier of the bio-cellulose film between the foam and the wound surface, wherein the bio-cellulose film has excellent anti-adhesion properties, therefore, the negative pressure wound dressing of the present application does not stick to the wound, and the new tissue does not grown into the foam, reducing the pain that the patient suffers when replacing the negative pressure wound dressing, and greatly decreasing the secondary damage to the wound. By leaving the drug in the pores of the foam and/or bio-cellulose film, the drug can be further released to the wound during the treatment, promoting the healing of the wound.

## Claims

1. A negative pressure wound dressing (1) comprising:
Foam (10); and
a bio-cellulose film (12) having a three-dimensional reticular structure (121), the bio-cellulose film (12) being bonded to a surface of the foam (10), with a projected area of the bio-cellulose film (12) on a surface of the foam (10) bonded to the bio-cellulose film (12) being greater than or equal to a projected area of the foam (10);
wherein the bio-cellulose film (12) comprises bio-celluloses secreted by bacteria and the bio-cellulose film (12) has a bio-cellulose content of 0.0013 g/cm² to 0.0018 g/cm² per unit area;
wherein the three-dimensional reticular structure (121) has a plurality of backbone fibers (121a) parallel to each other and a plurality of interlayered fibers (121b) interwoven with any two adjacent backbone fibers (121a).

2. The negative pressure wound dressing of claim 1, wherein the bacteria is at least one genus selected from the group consisting of *Gluconacetobacter, Acetobacter, Rhizobium, Sarcina, Pseudomonas, Achromobacter, Alcaligenes, Enterobacter, Azotobacter* and *Agrobacterium.*

3. The negative pressure wound dressing (1) of claim 1, wherein the backbone fibers (121a) and the interlayered fibers (121b) are made of bio-cellulose, and a diameter of the backbone fiber (121a) is greater than or equal to a diameter of the interlayered fiber (121b).

4. The negative pressure wound dressing (1) of claim 3, wherein the bio-cellulose has a diameter of 20 nm to 100 nm.

5. The negative pressure wound dressing (1) of claim 1, wherein the negative pressure wound dressing (1) further comprises a drug included in at least one of the foam (10) and the bio-cellulose film (12).

6. The negative pressure wound dressing (1) of claim 5, wherein the drug is at least one selected from the group consisting of an antibiotic, an antibacterial agent, a growth factor, fibrin, a vasodilator, an angiogenesis promoter, an antioxidant and an anti-inflammatory agent.

7. The negative pressure wound dressing (1) of claim 1, wherein the foam (10) comprises at least one material selected from the group consisting of polyurethane foam, polyvinyl alcohol foam, polyether foam, polyester foam, polylactic acid foam, polyolefin foam, chitosan foam, cellulose foam, alginate foam, gelatin foam and collagen foam.

8. The negative pressure wound dressing (1) of claim 1, wherein the foam has a single layer structure or a multilayer structure.

9. The negative pressure wound dressing (1) of claim 8, wherein the multilayer structure comprises a contact layer (301), an absorption layer (302) and a drainage layer (303) in sequence, and wherein the contact layer (301) is in contact with the bio-cellulose film (12).

10. A negative pressure wound treatment device, comprising:
the negative pressure wound dressing (1)of claim 1;
a liquid delivery tube (46) for connecting the negative pressure wound dressing (1); and
a negative pressure source (49).

11. The negative pressure wound treatment device of claim 10, further comprising a sealing film (45) disposed on the negative pressure wound dressing (1) for covering a wound (440) and a normal skin outside the wound (440).

12. The negative pressure wound treatment device of claim 11, wherein an opening (450) is formed in a center of the sealing film (45) and penetrates through two opposite surfaces of the sealing film (45), and one end of the liquid delivery tube (46) extends to the foam (10) of the negative pressure wound dressing (1) through the opening (450).

13. The negative pressure wound treatment device of claim 10, further comprising a liquid-collecting tank (47) for connecting the other end of the liquid delivery tube (46), and the liquid-collecting tank (47) is connected to the negative pressure source (49) through a connecting tube (48).

## Patentansprüche

1. Unterdruckwundauflage (1), aufweisend:
einen Schaumstoff (10); und
eine Bio-Cellulose-Folie (12) mit einer dreidimensionalen Netzstruktur (121), wobei die Bio-Cellulose-Folie (12) an eine Oberfläche des Schaumstoffs (10) gebunden ist, wobei eine projizierte Fläche der Bio-Cellulose-Folie (12) auf eine Oberfläche des Schaumstoffs (10), die an die Bio-Cellulose-Folie (12) gebunden ist, größer als oder gleich einer projizierten Fläche des Schaumstoffs (10) ist;
wobei die Bio-Cellulose-Folie (12) von Bakterien abgesonderte Bio-Cellulosen umfasst und die Bio-Cellulose-Folie (12) einen Bio-Cellulose-Gehalt von 0,0013 g/cm² bis 0,0018 g/cm² pro Flächeneinheit aufweist;
wobei die dreidimensionale Netzstruktur (121) eine Vielzahl von Rückgratfasern (121a) parallel zueinander und eine Vielzahl von Zwischenschichtfasern (121b) aufweist, die mit je zwei benachbarten Rückgratfasern (121a) verwoben sind.

2. Unterdruckwundauflage nach Anspruch 1, wobei es sich bei dem Bakterium um mindestens eine Gattung handelt, die ausgewählt ist aus der Gruppe, die aus *Gluconacetobacter, Acetobacter, Rhizobium, Sarcina, Pseudomonas, Achromobacter, Alcaligenes, Enterobacter, Azotobacter und Agrobacterium* besteht.

3. Unterdruckwundauflage (1) nach Anspruch 1, wobei die Rückgratfasern (121a) und die Zwischenschichtfasern (121b) aus Bio-Cellulose bestehen, und ein Durchmesser der Rückgratfaser (121a) größer als oder gleich einem Durchmesser der Zwischenschichtfaser (121b) ist.

4. Unterdruckwundauflage (1) nach Anspruch 3, wobei die Bio-Cellulose einen Durchmesser von 20 nm bis 100 nm hat.

5. Unterdruckwundauflage (1) nach Anspruch 1, wobei die Unterdruckwundauflage (1) darüber hinaus ein Medikament aufweist, das im Schaumstoff (10) und/oder in der Bio-Cellulose-Folie (12) enthalten ist.

6. Unterdruckwundauflage (1) nach Anspruch 5, wobei es sich bei dem Medikament um mindestens eines handelt, das aus der Gruppe ausgewählt ist, die aus einem Antibiotikum, einem antibakteriellen Mittel, einem Wachstumsfaktor, Fibrin, einem Vasodilatator, einem Angiogenese-Promotor, einem Antioxidans und einem entzündungshemmenden Mittel besteht.

7. Unterdruckwundauflage (1) nach Anspruch 1, wobei der Schaumstoff (10) mindestens ein Material aufweist, das aus der Gruppe ausgewählt ist, die aus Polyurethan-Schaumstoff, Polyvinylalkohol-Schaumstoff, Polyether-Schaumstoff, Polyester-Schaumstoff, Polymilchsäure-Schaumstoff, Polyolefin-Schaumstoff, Chitosan-Schaumstoff, Cellulose-Schaumstoff, Alginat-Schaumstoff, Gelatine-Schaumstoff und Kollagen-Schaumstoff besteht.

8. Unterdruckwundauflage (1) nach Anspruch 1, wobei der Schaumstoff eine Einschichtstruktur oder eine Mehrschichtstruktur aufweist.

9. Unterdruckwundauflage (1) nach Anspruch 8, wobei die Mehrschichtstruktur nacheinander eine Kontaktschicht (301), eine Absorptionsschicht (302) und eine Drainageschicht (303) umfasst, und wobei die Kontaktschicht (301) in Kontakt mit der Bio-Cellulose-Folie (12) steht.

10. Unterdruckwundbehandlungsvorrichtung, aufweisend:
die Unterdruckwundauflage (1) nach Anspruch 1;
ein Flüssigkeitszufuhrrohr (46) zum Anschließen der Unterdruckwundauflage (1); und
eine Unterdruckquelle (49).

11. Unterdruckwundbehandlungsvorrichtung nach Anspruch 10, darüber hinaus mit einer auf der Unterdruckwundauflage (1) angeordneten Dichtungsfolie (45) zur Abdeckung einer Wunde (440) und einer normalen Haut außerhalb der Wunde (440) .

12. Unterdruckwundbehandlungsvorrichtung nach Anspruch 11, wobei eine Öffnung (450) in einer Mitte der Dichtungsfolie (45) ausgebildet ist und durch zwei entgegengesetzte Oberflächen der Dichtungsfolie (45) hindurchgeht, und ein Ende des Flüssigkeitszufuhrrohrs (46) sich durch die Öffnung (450) zum Schaumstoff (10) der Unterdruckwundauflage (1) erstreckt.

13. Unterdruckwundbehandlungsvorrichtung nach Anspruch 10, darüber hinaus mit einem Flüssigkeitssammelbehälter (47) zum Anschließen des anderen Endes des Flüssigkeitszufuhrrohres (46), und wobei der Flüssigkeitssammelbehälter (47) über ein Verbindungsrohr (48) mit der Unterdruckquelle (49) verbunden ist.

## Revendications

1. Pansement par pression négative (1) comprenant :
de la mousse (10) ; et
un film de biocellulose (12) présentant une structure réticulaire tridimensionnelle (121), le film de biocellulose (12) étant lié à une surface de la mousse (10), une aire projetée du film de biocellulose (12) sur une surface de la mousse (10) liée au film de biocellulose (12) étant supérieure ou égale à une aire projetée de la mousse (10) ;
sachant que le film de biocellulose (12) comprend des biocelluloses sécrétées par des bactéries et le film de biocellulose (12) a une teneur en biocellulose de 0,0013 g/cm² à 0,0018 g/cm² par unité d'aire ;
sachant que la structure réticulaire tridimensionnelle (121) présente une pluralité de fibres dorsales (121a) parallèles les unes aux autres et une pluralité de fibres intercalaires (121b) entrelacées avec l'une quelconque de deux fibres dorsales (121a) adjacentes.

2. Le pansement par pression négative de la revendication 1, sachant que les bactéries sont au moins un genre sélectionné dans le groupe constitué par *Gluconacetobacter, Acetobacter, Rhizobium, Sarcina, Pseudomonas, Achromobacter, Alcaligenes, Enterobacter, Azotobacter* et *Agrobacterium.*

3. Le pansement par pression négative (1) de la revendication 1, sachant que les fibres dorsales (121a) et les fibres intercalaires (121b) sont composées de biocellulose, et un diamètre de la fibre dorsale (121a) est supérieur ou égal à un diamètre de la fibre intercalaire (121b).

4. Le pansement par pression négative (1) de la revendication 3, sachant que la biocellulose a un diamètre de 20 nm à 100 nm.

5. Le pansement par pression négative (1) de la revendication 1, sachant que le pansement par pression négative (1) comprend en outre un médicament inclus dans au moins l'un de la mousse (10) et du film de biocellulose (12).

6. Le pansement par pression négative (1) de la revendication 5, sachant que le médicament est au moins un médicament sélectionné dans le groupe constitué par un antibiotique, un agent antibactérien, un facteur de croissance, une fibrine, un vasodilatateur, un promoteur d'angiogenèse, un antioxydant et un agent anti-inflammatoire.

7. Le pansement par pression négative (1) de la revendication 1, sachant que la mousse (10) comprend au moins un matériau sélectionné dans le groupe constitué par de la mousse de polyuréthane, de la mousse d'alcool de polyvinyle, de la mousse de polyéther, de la mousse de polyester, de la mousse d'acide polylactique, de la mousse de polyoléfine, de la mousse de chitosane, de la mousse de cellulose, de la mousse d'alginate, de la mousse de gélatine et de la mousse de collagène.

8. Le pansement par pression négative (1) de la revendication 1, sachant que la mousse présente une structure monocouche ou une structure multicouche.

9. Le pansement par pression négative (1) de la revendication 8, sachant que la structure multicouche comprend une couche de contact (301), une couche d'absorption (302) et une couche de drainage (303) en séquence, et sachant que la couche de contact (301) est en contact avec le film de biocellulose (12).

10. Dispositif de traitement de plaie par pression négative, comprenant :
le pansement par pression négative (1) de la revendication 1 ;
un tube d'administration de liquide (46) destiné à se connecter au pansement par pression négative (1) ; et
une source de pression négative (49).

11. Le dispositif de traitement de plaie par pression négative de la revendication 10, comprenant en outre un film d'étanchéité (45) disposé sur le pansement par pression négative (1) pour couvrir une plaie (440) et une peau normale à l'extérieur de la plaie (440).

12. Le dispositif de traitement de plaie par pression négative de la revendication 11, sachant qu'une ouverture (450) est formée dans un centre du film d'étanchéité (45) et pénètre à travers deux surfaces opposées du film d'étanchéité (45), et une extrémité du tube d'administration de liquide (46) s'étend à la mousse (10) du pansement par pression négative (1) via l'ouverture (450).

13. Le dispositif de traitement de plaie par pression négative de la revendication 10, comprenant en outre un réservoir de collecte de liquide (47) destiné à se connecter à l'autre extrémité du tube d'administration de liquide (46), et le réservoir de collecte de liquide (47) est connecté à la source de pression négative (49) via un tube de connexion (48) .
